(19) **Europäisches Patentamt**
**European Patent Office**
**Office européen des brevets**

(11) **EP 3 928 764 B1**

(12) **EUROPEAN PATENT SPECIFICATION**

(45) Date of publication and mention
of the grant of the patent:
**31.07.2024 Bulletin 2024/31**

(21) Application number: **21180992.6**

(22) Date of filing: **22.06.2021**

(51) International Patent Classification (IPC):
*A61K 9/00* *(2006.01)*    *A61K 9/107* *(2006.01)*
*A61K 31/375* *(2006.01)*    *A61K 31/593* *(2006.01)*
*A61K 33/06* *(2006.01)*

(52) Cooperative Patent Classification (CPC):
(C-Sets available)
**A61K 9/009; A61K 9/0095; A61K 31/375;
A61K 31/593; A61K 33/06; A61K 33/10**    (Cont.)

(54) **ORAL FORMULATION BASED ON VITAMIN D3, ASCORBIC ACID, AND MAGNESIUM SALTS FOR USE AS A DIETARY SUPPLEMENT**

ORALE ZUSAMMENSETZUNG BASIERT AUF VITAMIN D3, ASCORBINSÄURE UND MAGNESIUM SALZEN ALS NAHRUNGSERGAENZUNGSMITTEL

COMPOSITION ORALE À BASE DE VITAMINE D3, D'ACIDE ASCORBIQUE ET DE SEL DE MAGNÉSIUM COMME COMPLÉMENT ALIMENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2020 IT 202000015007**

(43) Date of publication of application:
**29.12.2021 Bulletin 2021/52**

(73) Proprietor: **Bios Line S.p.A.**
**35020 Ponte San Nicolò (PD) (IT)**

(72) Inventor: **Tramonti, Paolo**
**35020 Ponte San Nicolò PD (IT)**

(74) Representative: **Perani & Partners S.p.A.**
**Piazza Armando Diaz, 7**
**20123 Milano (IT)**

(56) References cited:
**WO-A1-2020/026187**    **US-B2- 9 737 563**

- **ANONYMOUS: "Ultramag Magnesium - Product information sheet", PUREENCAPSULATIONS.COM, 1 March 2018 (2018-03-01), XP055554149, Retrieved from the Internet <URL:https://www.pureencapsulations.com/media/UltraMag%20Magnesium_1000-411-REVA.pdf> [retrieved on 20190208]**
- **WPI WORLD PATENT INFORMATION DERWENT, vol. 18, no. 83, 3 February 1983 (1983-02-03), XP002086534**

EP 3 928 764 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/375, A61K 2300/00;**
**A61K 31/593, A61K 2300/00;**
**A61K 33/06, A61K 2300/00;**
**A61K 33/10, A61K 2300/00**

**Description**

**FIELD OF THE INVENTION**

The field of the present invention relates to an oral formulation for use in replenishing magnesemia, maintaining muscle, osteo-articular, nervous, and immune function.

**BACKGROUND ART**

Food should provide all the nutrients necessary to maintain an adequate physiological condition of the body. Sometimes food alone is not enough to meet the needs of the body; for example, consider subjects suffering from diseases and/or allergies and/or food intolerances and/or subjects choosing to be vegan/vegetarian. In these and other cases, additional resources or supplements are required.

Vitamin $D_3$ (or cholecalciferol) is a steroid derived from dehydrocholesterol. Following exposure to ultraviolet radiation, it is the precursor to the biologically active form of vitamin D known as calcitriol. The latter promotes the physiological growth of the skeleton, bone remodelling, preventing the degeneration thereof with old age. Calcitriol also has other biological effects, including a role in cell growth, various neuromuscular and immune functions, and in reducing inflammation. It is known that vitamin $D_3$ is consumed only 10% with food. Age, dark skin, little time spent outdoors, the use of protective creams and low cholesterol levels are just some of the factors that affect the absorption of vitamin $D_3$ at the level of the first intestinal tract, and which prevent the biosynthesis thereof at the dermal level. A vitamin D deficiency can be associated with some acute and/or chronic diseases: periodontitis, rickets, osteomalacia, osteoporosis, autoimmune disorders, infectious diseases, cardiovascular diseases, type 2 diabetes, various types of cancer, and neurological disorders.

Vitamin C (or ascorbic acid) cannot be synthesized by the human body and, as such, must be introduced externally with the diet. Vitamin C plays an important role in immune function, as well as constituting a powerful natural antioxidant and being involved in the biosynthesis of the collagen of connective tissue. Vitamin C is predominantly absorbed at the intestinal level through active transport mechanisms or by means of facilitated glucose transport. Vitamin C deficiency can lead to pathological conditions such as scurvy.

Magnesium is an abundant mineral present in the human body which acts as a cofactor in more than 300 enzymatic systems; it can finely regulate various biochemical processes, such as protein synthesis, nerve and muscle function, control of blood glucose levels and blood pressure, energy production in the field of oxidative phosphorylation. Furthermore, it is effective in activating vitamin D from vitamin $D_3$, and thus in bone development.

WO2020/026187 A1 relates to a unitary oral composition for use in the control of mild to moderate arterial hypertension comprising: *Beta vulgaris* dry extract, magnesium oxide, ascorbic acid and optionally at least one of the vitamins selected from vitamin $B_1$ and vitamin $D_3$, in which the ascorbic acid and magnesium oxide are in a molar ratio between a minimum of 0.25:1 and a maximum of 1:2. Anonymous: "Ultramag Magnesium - Product information sheet",Pureencapsulations.com, 1 March 2018 (2018-03-01), XP055554149,Retrieved from the Internet:URL:https://www.pureen-capsulations.com/media/ UltraMag%20Magnesium_1000-411-REVA.pdf describes a capsule using microencapsulation technology to enhance the absorption of magnesium to promote musculoskeletal, cardiometabolic and emotional health. It contains magnesium (as Sucrosomial® magnesium) and pregelatinized rice starch, sucrose esters of fatty acids, vegetarian capsule (cellulose, water), hypoallergenic plant fiber (cellulose), sunflower lecithin, tricalcium phosphate, rice flour.

*Problems of the prior art*

The intake of vitamin $D_3$, vitamin C and magnesium $Mg^{2+}$ in the form of the salts thereof can be compromised in the intestinal system, especially when the body suffers from pathological conditions, intolerances and/or food allergies. In particular, it is known that:

- the absorption of vitamin $D_3$ in the small intestine is low and can be influenced by the presence of other liposoluble substances, such as long chain fatty acids, with the establishment of competitive mechanisms;
- vitamin C (or ascorbic acid) in aqueous solution is highly unstable due to the oxidation reaction with oxygen and the consequent formation of dehydroascorbic acid;
- magnesium ascorbate is a hygroscopic salt which is difficult to find on the market.

**SUMMARY OF THE INVENTION**

The Applicant has developed an oral formulation for use in replenishing magnesemia, maintaining muscle, osteo-articular, nervous and immune function comprising: ascorbic acid, vitamin $D_3$ and magnesium salts, in which the vitamin $D_3$ is conveyed in the form of micellar dispersion, the magnesium salts contain at least magnesium carbonate and magnesium oxide, and the weight ratio between ascorbic acid to magnesium salts is comprised between 0.2:1 and 1.25:1.

*Advantages of the invention*

With respect to the prior art, the Applicant has now developed an oral formulation which is advantageous because:

- it allows to act *in parallel* on different fronts: muscle, osteo-articular, nervous and immune, contributing to the maintenance of the physiological state of the body, with three different active ingredients: vitamin $D_3$, vitamin C and magnesium salts, specifically magnesium carbonate and magnesium oxide;

- the bioavailability of vitamin $D_3$ in the small intestine is improved by the formation of the micellar dispersion with fatty acid sucrose esters;

- the ascorbic acid is in an anhydrous powder form and as such is more stable than the chemical form thereof in aqueous solution;

- magnesium ascorbate is formed *in situ* in the gastric tract, from ascorbic acid and magnesium carbonate and magnesium oxide. Increasing the pH of the gastric environment - up to a pH value of between 4 and 5 - and the in *situ* formation of magnesium ascorbate allow to advantageously increase the bioavailability of magnesium in the small intestine. Furthermore, magnesium ascorbate is known to be a hygroscopic salt. Therefore, the generation thereof *in situ* avoids, on the one hand, any problems of preparation of the formulation in final powder form and, on the other hand, the risk of compromising the chemical-physical stability of said formulation;

- in addition to magnesium ascorbate and magnesium oxide, the formulation contains other resources of magnesium $Mg^{2+}$ such as magnesium pidolate and magnesium citrate. In scientific terms, the co-presence of different sources of magnesium is thought to increase the amount which is absorbable by the body. Magnesium $Mg^{2+}$ also promotes the activation of the vitamin $D_3$ simultaneously present in the claimed formulation;

- the bioavailability of magnesium ($Mg^{2+}$) comprised in the oral formulation object of the invention is increased (see Example 5). In this regard, the Applicant believes that it is precisely the presence of all the active ingredients comprised in the oral formulation for the purposes of the invention, especially the vitamin $D_3$ conveyed in the form of micellar dispersion, which favours an increase in the bioavailability of magnesium.

**DESCRIPTION OF THE FIGURES**

**Figure** 1: Action mechanism of the oral formulation.

**Figure 2:** Image representative of the determination of $t_{max}$.

**Figure 3:** Image representative of in vitro intestinal epithelium model with Caco-2 cell monolayer grown on a Transwell® insert.

**Figure 4:** Relative bioaccessibility of magnesium, expressed as a percentage (%) of total magnesium at the end of the digestive process (see Example 5, section 5.3.3).

**Figure 5:** Impact of increasing concentrations of the bioaccessible fraction of the tested "complete magnesium" comparison formulation on intestinal mucosal viability, assessed by MTS assay (the symbol * indicates less than 70%) (see Example 5, section 5.3.4).

**Figure 6:** Impact of increasing concentrations of the bioaccessible fraction of the tested Example 4 formulation on intestinal mucosal viability, assessed by MTS assay (the symbol * indicates less than 70%) (see Example 5, section 5.3.4).

**Figure 7**: Bioavailability of magnesium expressed as a percentage (%) after 3 hours of exposure of the intestinal epithelium model in *vitro* to the maximum non-toxic concentration of the bioaccessible fraction of the "complete magnesium" formulation and Example 4 (p<0.05) (see Example 5, section 5.3.5).

**Figure 8:** Apparent permeability after 3 hours of exposure of the intestinal epithelium model in *vitro* to the maximum non-toxic concentration of the bioaccessible fraction of "complete magnesium" formulation and of Example 4 (p<0.05). (see Example 5, section 5.3.5).

**Figure 9:** Cellular viability of the intestinal mucosa following treatment for 3 hours with digestive fluids (FD) and the maximum non-toxic concentration of the bioaccessible fraction of the "complete magnesium" comparison and Example 4 formulations based on magnesium (see Example 5, section 5.3.6).

## DETAILED DESCRIPTION OF THE INVENTION

In pharmacology, bioavailability is the fraction of drug administered which reaches systemic circulation (or blood circulation) without undergoing any chemical modification with respect to the total administered.

Maintenance of muscular, osteo-articular and immune function means the ability, on the one hand, to preserve the structural integrity and physiological functioning of the muscular, nervous and osteo-articular system, and to contribute to the support of immune defence; and on the other hand, to act by restoring physiological function in altered or pathological conditions of the muscular, osteo-articular, nervous and immune systems. For example, it is scientifically believed that the active ingredients of said formulation promote homoeostasis and the absorption of minerals such as calcium and phosphate; they act on the inhibition of osteoblasts, regulate extracellular matrix mineralization and the biosynthesis of pro-collagen and carnitine; they protect the epithelial barrier against pathogens, promote chemotaxis, phagocytosis, the generation of reactive oxygen species, the differentiation and proliferation of B and T cells. The maintenance of muscle, osteo-articular, nervous and immune function may be required, for example, in the case of myalgia, osteo-tendon and joint inflammation, osteoarthritis, autoimmune disorders and/or immunodeficiency states.

It should be noted that the formulation can also be indicated for the treatment of irritability, nervousness, excessive stress, mood swings, fatigue or tiredness.

These disorders are often associated with a magnesium deficiency: a very important mineral because it is involved in processes which are fundamental to the health and balance of our body. In fact, magnesium contributes to the normal functioning of the nervous system.

Magnesium is normally available in many foods, but an unbalanced diet, incorrect cooking, or particular phenomena such as sports activity, taking some drugs or physiological conditions such as pregnancy, breastfeeding and the pre-menstrual period can lead to a deficiency situation. In these cases, the dietary supplementation of magnesium is particularly useful.

According to a further embodiment, the formulation of the invention is intended for use in the replenishment of magnesemia, e.g., in deficiency states (hypomagnesemia).

As already mentioned, the level of magnesium is lowered due to a reduction in magnesium in the diet (most often due to malnutrition) or due to the inability of the intestine to normally absorb nutrients (malabsorption). Other times, however, hypomagnesemia develops due to an excessive excretion of magnesium from the kidneys or intestine. Hypomagnesemia may also be caused by the following:

Consumption of large amounts of alcohol (common), which reduces the consumption of food (and therefore of magnesium) and increases the excretion of magnesium;

Prolonged diarrhoea (common), which increases the excretion of magnesium;

High levels of aldosterone, vasopressin (antidiuretic hormone) or thyroid hormones, which result in the increased excretion of magnesium;

Drugs which increase the excretion of magnesium, including diuretics, the antifungal drug amphotericin B and the chemotherapeutic drug cisplatin;

Chronic use of a proton pump inhibitor (drugs which reduce gastric acidity);

Hypomagnesemia can cause nausea, vomiting, drowsiness, weakness, personality changes, muscle spasms, tremors, and loss of appetite. In severe forms, hypomagnesemia can cause seizures, especially in children.

Preferably, the magnesium salts comprised in the oral formulation according to the invention are not in micellar or micellar dispersion form.

Together with the magnesium salts, the formulation comprises ascorbic acid, preferably used in the *L-ascorbic* acid form thereof.

The oral formulation contains vitamin $D_3$ conveyed in the form of micellar dispersion; said micellar dispersion preferably comprises fatty acid sucrose esters and preferably consists of fatty acid sucrose esters.

Preferably, said fatty acid sucrose esters are selected from the group consisting of: oleic acid sucrose esters, palmitic acid sucrose esters and mixtures thereof.

Said fatty acid sucrose esters are commonly used as food additives belonging to food categories E473 and/or E474 according to Regulation (EC) No. 1333/2008.

The oral formulation contains vitamin $D_3$ preferably in amounts ranging from 0.000015 g to 0.005 g, preferably 0.000020 g to 0.005 g, preferably ranging from 0.000036 g to 0.005 g, preferably ranging from 0.000036 g to 0.002 g.

The micellar dispersion of vitamin $D_3$ promotes the increased bioavailability thereof in the first intestinal tract (or small intestine).

Micellar dispersion means a dispersion in which the dispersed phase assumes micellar organizational structures, i.e., aggregates of molecules are formed with polar or ionic parts and non-polar parts; mainly the polar parts are distributed outwards while the non-polar parts inside.

Preferably, the magnesium salts further comprise a magnesium salt selected from magnesium pidolate, magnesium citrate and mixtures thereof.

Preferably said oral formulation contains all four magnesium salts: magnesium carbonate, magnesium oxide, magnesium pidolate and magnesium citrate.

It should be noted that according to a preferred embodiment, the magnesium oxide is in the form of marine magnesium, one of the most common forms of magnesium, obtained by the evaporation of seawater. Marine magnesium generally has a high concentration of elemental magnesium: 50%-60%, versus 12-15% for forms such as magnesium chloride or magnesium citrate.

In said oral formulation the magnesium salts are preferably in amounts between 0.240 g and 1.045 g.

Preferably, the magnesium carbonate is in amounts ranging from 0.150 g to 0.870 g, preferably ranging from 0.150 g to 0.850 g, preferably ranging from 0.150 g to 0.500 g, preferably ranging from 0.150 g to 0.300 g, preferably ranging from 0.150 g to 0.250 g.

Preferably, the magnesium oxide is in amounts ranging from 0.020 g to 0.100 g, preferably ranging from 0.040 g to 0.070 g.

Preferably, the magnesium pidolate and the magnesium citrate are each in amounts ranging from 0.0040 g to 0.700 g, preferably ranging from 0.0060 g to 0.700 g, preferably ranging from 0.0080 to 0.700, preferably ranging from 0.100 g to 0.700 g.

In the oral formulation the ascorbic acid is preferably in amounts ranging from 0.0500 g to 0.300 g, preferably ranging from 0.100 to 0.300 g, preferably ranging from 0.200 g to 0.300 g, preferably ranging from 0.250 g.

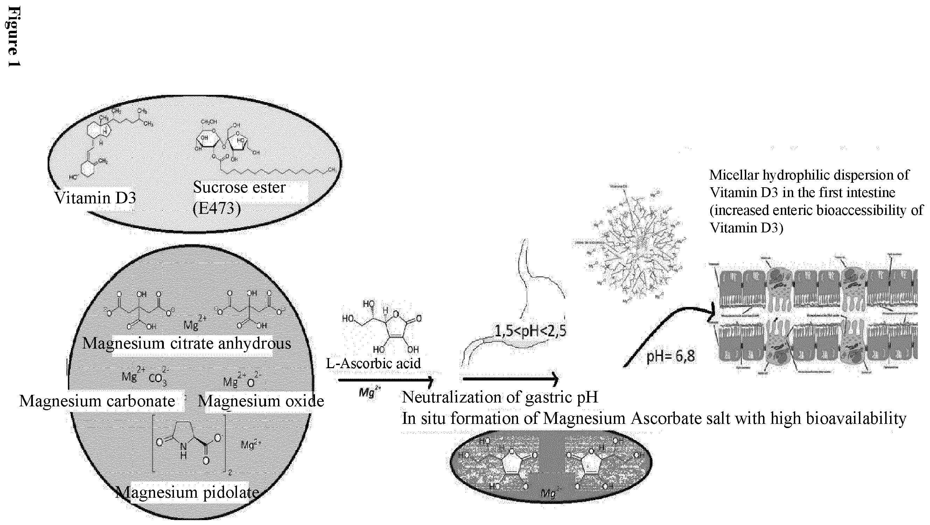

The presence, in the formulation, of at least magnesium carbonate and magnesium oxide allows the formation of magnesium ascorbate within the gastric environment (*in situ*). The magnesium oxide forms, by hydration, magnesium hydroxide which, by raising the gastric pH to values between 4 and 5, allows the local formation of magnesium ascorbate salt. This phenomenon favours the increase in bioavailability of magnesium ascorbate predominantly in the small intestine tract, at pH values equal to about 6.8 (Figure 1). Also the Magnesium Carbonate, following the interaction with the gastric hydrochloric acid, releases divalent magnesium ions which contribute to the formation of magnesium ascorbate salt, even in the absence of a contribution to alkalinization.

Preferably the oral formulation is in a water-dispersible powder form.

When the oral formulation is in the form of a water-dispersible powder, preferably the daily dosage is in amounts ranging from 1.40 g to 5.0 g, preferably ranging from 1.40 to 2.5 g, preferably 2.50 g.

The oral formulation can be formulated in the form of single-dose sachets or bottles, preferably provided with a powder dosing device.

## EXAMPLES

For illustrative and not limiting purposes, the Applicant lists below embodiment examples of the invented formulation, and the relative preparation method.

### *Example 1: Oral formulation (single-dose)*

| Phase | Components | gr |
|---|---|---|
| A | Magnesium Citrate Anhydrous (16% Mg) | 0.625 |
| A | Magnesium Carbonate (45% MgO) | 0.200 |
| A | Marine Magnesium (55% Mg) | 0.044 |
| A | Magnesium Pidolate (8.66% Mg) | 0.174 |
| A | L-Ascorbic Acid | 0.250 |
| A | Citric acid anhydrous (for powders) | 0.400 |
| A | FOS | 0.500 |
| B | SUCROSE ESTER E 476 | 0.003 |
| B | Vitamin D3 (1,000,000 IU/gr) | 0.000036 |
| C | Precipitated amorphous silica E | 0.050 |
| C | Stevia Rebaudiana (sweetener) | as needed |
| Total Magnesium provided: 400 mg<br>Total Vitamin $D_3$ provided: 28 UI | | |

### Example 2: Oral formulation (single-dose)

| Phase | Components | gr |
|---|---|---|
| A | Magnesium Citrate Anhydrous (16% Mg) | 0.625 |
| A | Magnesium Carbonate (45% MgO) | 0.200 |
| A | Marine Magnesium (55% Mg) | 0.044 |
| A | Magnesium Pidolate (8.66% Mg) | 0.174 |
| A | L-Ascorbic Acid | 0.250 |
| A | Citric acid anhydrous (for powders) | 0.400 |
| A | FOS | 0.500 |
| B | SUCROSE ESTER E 476 | 0.015 |
| B | Vitamin D3 (1,000,000 IU/gr) | 0.001 |
| C | Precipitated amorphous silica E | 0.050 |
| C | Stevia Rebaudiana (sweetener) | as needed |
| Total Magnesium provided: 400 mg<br>Total Vitamin $D_3$ provided: 1000 UI | | |

### Example 3: Preparation method of the oral formulation in Example 1 and 2

Combine, one at a time, the powders of Phase A by mixing with a bag or with a V mixer for at least 5 minutes. Sieve with mesh size 80 mesh and stir for another 5 minutes.

Prepare Phase B by moistening the sucrose ester with Vitamin $D_3$. Add Phase B to Phase A under constant mechanical stirring until a homogeneous powder is obtained and then sieve again. Finally, combine Phase C and stir again until homogeneous.

*Example 4: Bulk oral formulation to be dosed with dosing spoon*

| Components | Amount (g/100g) |
|---|---|
| Citric acid | 49.901 |
| Magnesium carbonate | 34.030 |
| **Vitamin C** | 7.560 |
| FOS - Fructooligosaccharides | 2.050 |
| Flavouring | 2.010 |
| Magnesium citrate | 1.780 |
| Marine magnesium | 1.613 |
| Magnesium L-pidolate | 0.323 |
| Steviol glycosides | 0.322 |
| Silicon dioxide | 0.290 |
| **Fatty acid sucrose esters** | 0.120 |
| **Cholecalciferol/Vitamin D$_3$** | 0.00144 |
| Total | 100.000 |

*Example 5: Comparative assessment study*

The purpose of the study is to evaluate and compare the solubility kinetics, bioaccessibility and bioavailability of two magnesium (Mg) formulations, namely the oral formulation of Example 4 and the "complete magnesium" oral comparison formulation below, the latter not being the subject of the present invention.

*"Complete magnesium" oral comparison formulation* (not the subject of the present invention).

| Components | Amount (g/100g) |
|---|---|
| Citric Acid | 57.582 |
| Magnesium carbonate | 34.030 |
| FOS - Fructooligosaccharides | 2.050 |
| Flavouring | 2.010 |
| Magnesium citrate | 1.780 |
| Marine magnesium | 1.613 |
| Magnesium L-pidolate | 0.323 |
| Steviol glycosides | 0.322 |
| Silicon dioxide | 0.290 |
| **Total** | **100.000** |

It should be noted that, with respect to the oral formulation of Example 4, the aforementioned "complete magnesium" oral comparison formulation does not comprise fatty acid sucrose esters nor Cholecalciferol/Vitamin D$_3$, nor Ascorbic acid.

**5.1 Materials and Methods**

The comparative assessment of magnesium solubility kinetics and intestinal absorption, by means of intestinal epithelium cell model, was performed considering the two oral formulations of Example 4 and the "complete magnesium" comparison.

*5.1.1 Determination of the magnesium content in the formulations*

The magnesium content in the formulations was determined by inductively coupled plasma mass spectrometry (ICP-MS) (NexIon 300D, PerkinElmer). The samples (10 mg of Example 4 and "complete magnesium" comparison formulations or 0.5 mL of liquid samples) were transferred to centrifuge tubes and 1 mL of nitric acid was added. The samples were then kept for 8 hours at 70°C and, at the end of digestion, the digest was transferred to a 50 mL centrifuge tube and diluted to a final volume of 50 mL with ultra-pure water (ddH2O or "double distilled water"). The basolaterial samples (intestinal absorption) were not digested but centrifuged at 16900 g for 10 minutes and subsequently diluted. Three independent replicates for each sample were prepared and analysed by ICP-MS.

*5.1.2 Assessment of the solubility kinetics of the formulations of Example 4 and "complete magnesium " comparison*

The solubility kinetics of the formulations of Example 4 and the magnesium-based "complete magnesium" comparison formulation, respectively, were evaluated by (i) a turbidimetric approach and (ii) a dialysis approach, discussed in detail in the next two paragraphs.

*5.1.2.1 Turbidimetric approach*

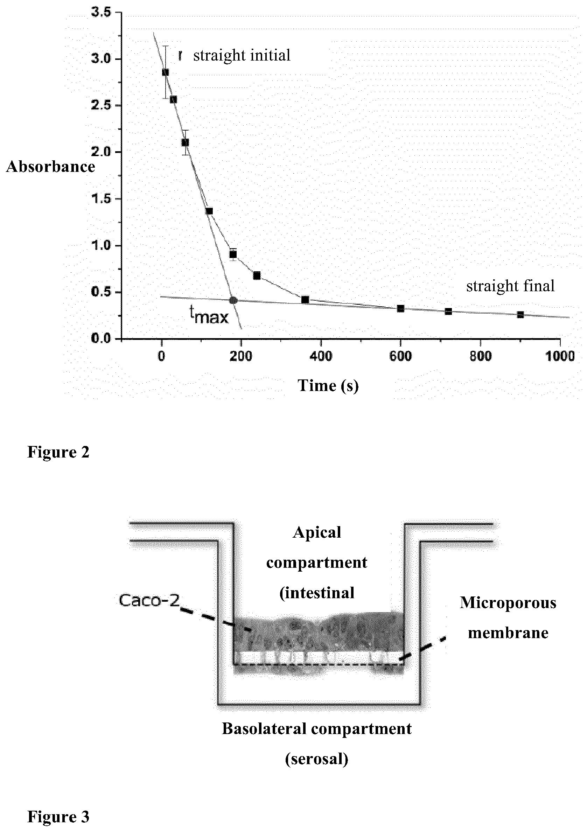

2.5 g and 5 g of both the "complete magnesium" comparison and Example 4 magnesium-based formulations were suspended in 200 mL ddH$_2$O and kept under constant stirring at room temperature. Subsequently, at defined times (0, 10, 30, 60, 120, 180, 240, 360, 600, 720 and 900 seconds), an aliquot of the suspension was taken and the absorbance thereof (turbidity) was measured spectrophotometrically at 600 nm. Tmax is defined as the time at which the dissolution of the formulation is maximum; Tmax was calculated for each formulation as the intersection point of the lines obtained from the linear fit of the initial and final phase of the solubility kinetics curves (Figure 2).
The comparison between the two formulations was calculated using the following formula:

$$Relative\ solubility\ (\%) = 100 - \left( \left( \frac{Tmax\ of\ Example\ 4\ formulation}{Tmax\ of\ \text{"complete magnesium"}\ formulation} \right) \times 100 \right)$$

whereby a positive value of the relative solubility indicates that the formulation of Example 4 is more soluble than the "complete magnesium" comparison formulation, while a negative value indicates the opposite.

*5.1.2.2 Dialysis*

2.5 g and 5.0 g of the formulation of Example 4 and the "complete magnesium" comparison formulation were dissolved in 200 mL water (equivalent to 1.15 and 2.30 mg/mL Mg, respectively). 0.9 and 1.8 mg Mg for each magnesium-based formulation were dialysed against ddH2O (6-8 kDa dialysis membrane cut-off). As with the turbidimetric approach, aliquots of the dialysis buffer were collected at defined times (0, 10, 30, 60, 120, 180, 240, 360, 600, 720, 900, 1200 and 1800 s) and the magnesium content thereof determined by means of ICP-MS. In light of the results of the turbidimetric approach and the objective of the formulations, the dissolution kinetics were analysed within 3 minutes. The obtained data were linearly fitted, according to the following equation:

$$Mg_i = m \times t_i + q$$

in which $Mg_i$ is the amount of magnesium at time *i, m* the slope of the line, $t_i$ the time and q the intercept of the line resulting from the linear fit.
To compare the solubility of the formulation of Example 4 with that of the "complete magnesium" comparison, the following ratio was considered:

$$Relative\ solubility\ = \frac{m\ Example\ 4\ formulation}{m\ \text{"complete magnesium"}\ formulation}$$

whereby a relative solubility value > 1 indicates that the formulation of Example 4 is more soluble than the "complete magnesium" comparison formulation, and a value < 1 the opposite.

### 5.1.3 Digestive process

2.5 g of formulation of Example 4 and the "complete magnesium" comparison were subjected to the *in vitro* digestion procedure, developed to simulate the human digestive process (oral, gastric and intestinal compartments). To avoid any possible contamination and interference, the Mg contributions from the simulating digestive fluids and digestive process were carefully removed. At the end of the digestive process, the magnesium concentration was measured in the complete digests and compared to the applied dose to estimate the overall recovery of the process. After centrifugation at 2750 g for 5 min, the magnesium was quantified in the supernatants corresponding to the bioaccessible fraction by means of ICP-MS.

### 5.1.4 In vitro intestinal epithelium model

The intestinal absorption of the magnesium present in the "complete magnesium" comparison and Example 4 formulations was determined using an *in vitro* human intestinal model based on human Caco-2 intestinal adenocarcinoma cells (ATCC, HTB-37™) organized as a functional monolayer on commercially available Transwell®-type inserts. Transwell® inserts feature two compartments separated by a microporous membrane: apical (or luminal) and basolateral (or serosal). The Caco-2 monolayers grown on the microporous membranes consist of polarized cells with morpho-functional features typical of enterocytes, such as the presence of microvilli, tight junctions and P-glycoprotein (P-gp) (Figure 3).

### 5.1.5 Assessment of the impact of the digested formulations on intestinal epithelial viability

In order to assess the impact of the digested formulations on intestinal epithelial viability, the bioaccessible fraction of the tested formulations was serially diluted in digestive fluids. The resulting bioaccessible fraction concentrations were added to the apical side of the intestinal model in vitro, while magnesium-free HBSS (Hanks' Balanced Salt Solution) was added to the basolateral compartment. Digestive fluids, without formulations, were used as a negative control. After 3 hours of incubation, the viability of the intestinal epithelia was assessed by means of MTS assay, based on the reduction of the MTS tetrazolium compound by cells vital to generate the coloured formazan product, quantifiable by measuring the absorbance thereof at 490 nm. As a result, the cell viability is directly proportional to the absorbance. The bioavailability experiments were performed using the maximum non-toxic concentration of the two formulations, as determined by the dose-response curves. The results obtained are expressed as a percentage (%) with respect to the negative control.

### 5.1.6 Assessment of the intestinal absorption of magnesium

At the end of the digestive process, the maximum non-toxic concentration of the bioaccessible (supernatant) fraction of the tested formulations of Example 4 and "complete magnesium" comparison was added to the apical compartment of the intestinal epithelium *in vitro,* while magnesium-free HBSS was added to the basolateral compartment. After 3 hours of exposure, the apical and basolateral fractions were recovered and the magnesium content thereof was determined by means of IPC-MS analysis.

The bioavailability of the magnesium was calculated and expressed as: absorbed amount ($\mu$g), as percentage of absorption (%) with respect to the amount of Mg added to the apical compartment at the start of the absorption experiment, and as apparent permeability ($P_{app}$).

The relative permeability was also assessed by the following formula:

$$Relative\ Permeability = \frac{Papp\ Example\ 4\ formulation}{Papp\ "complete\ magnesium"\ formulation}$$

whereby a relative permeability value > 1 indicates that the formulation of Example 4 is more bioavailable than the "complete magnesium" formulation, and a value < 1 the opposite.

### 5.1.7 Viability and integrity of the intestinal epithelial model barrier

At the end of absorption, the cellular viability and barrier integrity of the intestinal epithelium model were assessed. The cell viability was assessed using the MTS assay. The results were expressed as a percentage (%) with respect to the negative control (intestinal epithelium treated with formulation-free digestive fluids).

**5.2 Statistical analysis**

All the data are presented as mean ± standard deviations (SD) of three independent experiments. To determine if there were statistically significant differences between the treatments, a T-test analysis was performed. The T-test is a statistical method used to test the differences between the means of two series. The differences between the groups were considered significant at p < 0.05. All the statistical analyses were performed with OriginLab software.

**5.3 RESULTS**

*5.3.1 Determination of magnesium titre in the tested formulations*

As reported in Table 1, the concentration of magnesium measured in the formulations "complete magnesium" and Example 4 is compatible with that declared, with a recovery of approximately 100% for both formulations.

Table 1 - Magnesium concentration in the tested formulations, expressed as a percentage (%, grams of Mg in 100 grams of formulation). The measured values are shown as mean ± standard deviation.

| | Concentration Nominal Mg (%) | Concentration Measured Mg (%) | Recovery (%) |
|---|---|---|---|
| "Complete magnesium" formulation | 9 | 9.0 ± 0.5 | 100.2 ± 6.1 |
| Example 4 Formulation | 9 | 9.8 ± 1.5 | 108.6 ± 6.1 |

*5.3.2 Solubility kinetics of the formulations of Example 4 and the "complete magnesium" comparison*

The solubility of a dietary supplement is essential to determine the beneficial effects thereof on the consumer. Many active substances are not effective due to the poor solubility and/or slow solubility kinetics thereof, which limits the bioaccessibility and bioavailability thereof. The solubility kinetics of the Example 4 and "complete magnesium" comparison formulations were analysed by two different approaches: (i) turbidimetric and (ii) dialysis (as specified in paragraphs 5.1.2.1 and 5.1.2.2 respectively).

Applying the turbidimetric test, the formulation of Example 4 shows a lower Tmax than that of the "complete magnesium" formulation at both amounts of Mg considered (145.2 s vs 169.2 s with 225 mg Mg, and 188.8 s vs 209.5 s with 450 mg Mg), indicating that the formulation of Example 4 is characterized by a higher relative solubility with respect to the "complete magnesium" formulation (14.1% and 10.2% for 225 and 450 mg Mg respectively) (Table 2).

Table 2 - Values of Tmax and relative solubility obtained following the analysis of the solubility kinetics data of the two magnesium-based formulations, up to 900 s and starting from 225 and 450 mg of Mg. A positive relative solubility value indicates that the formulation of Example 4 is more soluble than the "complete magnesium" comparison formulation, while a negative value indicates the opposite.

| Formulation | Mg (mg) | Tmax (s) | Relative Solubility (%) |
|---|---|---|---|
| "Complete magnesium" formulation | 225 | 169.2 | 14.1 |
| Example Formulation 4 | | 145.2 | |
| "Complete magnesium" formulation | 450 | 209.5 | 10.2 |
| Example Formulation 4 | | 188.3 | |

A similar trend was observed by testing the solubility of the formulations by means of dialysis. After exposure to dialysis of 12.5 mg formulation per mL ddH$_2$O, corresponding to 2500 mg formulation in 200 mL ddH2O, the dissolution rate during the first 3 minutes of incubation is 0.3532 and 0.2555 for the formulation of Example 4 and the "complete magnesium" comparison, respectively. With a dissolution rate of 0.6605 and 0.6325, the same trend was observed for the formulation of Example 4 and for the "complete magnesium" formulation when used at the concentration of 25 mg formulation/mL, corresponding to 5000 mg formulation in 200 mL ddH2O (Table 3).

Considering the overall results, the solubility of the magnesium Mg in the formulation of Example 4 is higher than that in the "complete magnesium" formulation.

Table 3 - Dissolution rate (m) and relative solubility (m formulation of Example 4 / m "complete magnesium" comparison formulation) obtained by dialysis with 225 and 450 mg Mg. Relative solubility values > 1 indicate that the formulation of Example 4 is more soluble than the "complete magnesium" comparison formulation; a value < 1 indicates the opposite.

| Formulation | Mg (mg) | m | Relative Solubility |
|---|---|---|---|
| "Complete magnesium" formulation | 0.9 | 0.2555 | 1.38 |
| Example Formulation 4 | | 0.3532 | |
| "Complete magnesium" formulation | 1.8 | 0.6326 | 1.04 |
| Example Formulation 4 | | 0.6605 | |

### 5.3.3 Magnesium release from the two formulations - bioaccessible fraction

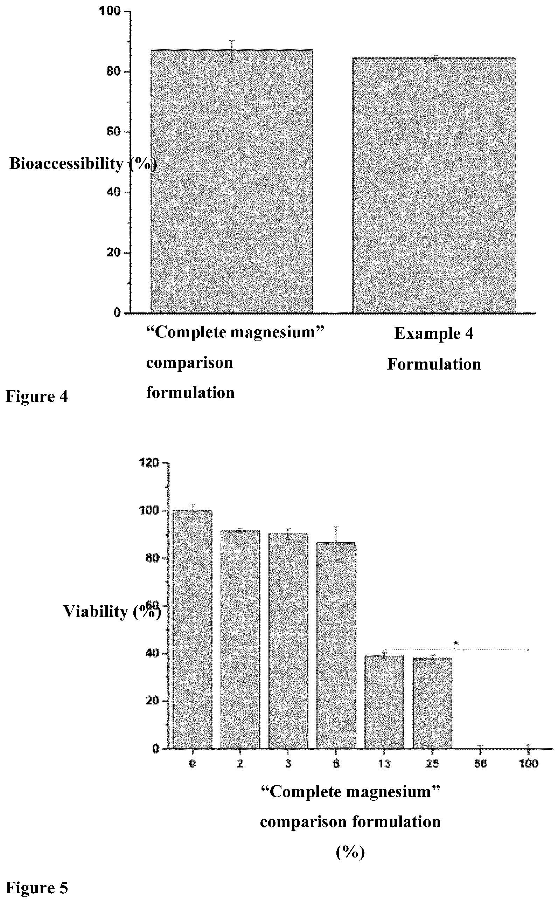

The bioaccessibility indicates the fraction of active ingredient released from the matrix into the gastrointestinal tract and available for absorption. To determine the bioaccessibility of the Mg, 2.5 g of each formulation (equivalent to a nominal amount of Mg of 225 mg) was exposed to in vitro digestive process simulating an adult man. At the end of the digestive process, the total amount of digested Mg and bioaccessible fractions were measured. While the "complete magnesium" oral comparison formulation has a total amount of Mg of $235.5 \pm 10.6$ mg and a bioaccessible fraction of $205.3 \pm 7.5$ (87.2%), the oral formulation of Example 4 has a total amount of Mg of $167.3 \pm 5.4$ mg and a bioaccessible fraction of $141.2 \pm 1.4$ (84.5%) (Figure 4 and Table 4).

Table 4 - Bioaccessible fraction of magnesium of the two formulations of Example 4 and "complete magnesium" comparison, expressed as mg and percentage (%) with respect to the amount of total magnesium measured at the end of the digestive process. The results are expressed as mean $\pm$ standard deviation.

| | Bioaccessibility | |
|---|---|---|
| | Mg (mg) | Mg (%) |
| "Complete magnesium" formulation | $205.3 \pm 7.5$ | $87.2 \pm 3.2$ |
| Example 4 Formulation | $141.2 \pm 1.4$ | $84.5 \pm 0.8$ |

### 5.3.4 Impact of the digested Example 4 and "complete magnesium" comparison formulations on intestinal epithelial viability

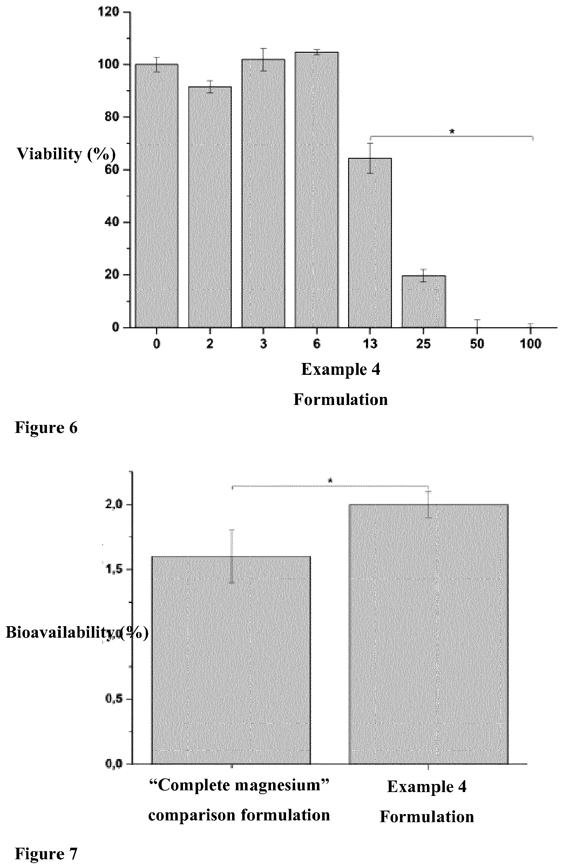

Before measuring the bioavailability of the magnesium, the impact of bioaccessible fractions of the two formulations on intestinal mucosal viability was evaluated. Caco-2 monolayers were exposed for 3 hours to increasing concentrations of bioaccessible fractions (from 0 to 100%), yielding dose-response curves (Figure 5, Figure 6 and Table 5). Both of the magnesium formulations significantly reduce the viability of Caco-2 monolayers starting from 13% concentration of the bioaccessible fraction (viability of Caco-2 monolayer < 70%) (Figure 5, Figure 6 and Table 5). As a result, the intestinal absorption studies were performed by exposing intestinal epithelia to diluted bioaccessible fractions (6%) ($86.4 \pm 7.1\%$ and $104.7 \pm 1.0\%$ viability for "complete magnesium" and Example 4 formulation respectively) (Figure 5, Figure 6 and Table 5).

Table 5 - Viability of the intestinal epithelium model in vitro following exposure to increasing concentrations of the bioaccessible fraction of the formulations. The results are shown as mean $\pm$ standard deviation.

| Concentration (%) | "Complete magnesium" formulation | Example 4 Formulation |
|---|---|---|
| 0 | $100.0 \pm 2.8$ | $100.0 \pm 2.8$ |
| 2 | $91.5 \pm 1.0$ | $91.5 \pm 2.4$ |
| 3 | $90.3 \pm 2.1$ | $101.9 \pm 4.3$ |

(continued)

| Concentration (%) | "Complete magnesium" formulation | Example 4 Formulation |
|---|---|---|
| 6 | 86.4 ± 7.1 | 104.7 ± 1.0 |
| 13 | 38.9 ± 1.3 | 64.4 ± 5.7 |
| 25 | 37.8 ± 1.9 | 19.6 ± 2.4 |
| 50 | 0.0 ± 1.5 | 0.0 ± 3.0 |
| 100 | 0.0 ± 2.0 | 0.0 ± 1.6 |

### *5.3.6 Magnesium bioavailability of Example 4 and "complete magnesium" comparison formulations*

To assess the bioavailability of magnesium released from the two formulations, *in vitro* intestinal epithelia were exposed for 3 hours to diluted bioaccessible fractions (6%), and the magnesium content was measured in both the apical (luminal) and basolateral (serosal) compartments. The bioavailability, i.e., the amount of Mg in the basolateral/serosal compartment, was then calculated and expressed as amount ($\mu$g), as percentage absorption (%) with respect to the amount of Mg initially added to the apical compartment, and as apparent permeability ($P_{app}$). As shown in Figure 7 and Table 6, the formulation of Example 4 shows a significantly higher bioavailability than the "complete magnesium" formulation (2.0 ± 0.1% vs 1.6 ± 0.2% bioavailability respectively, equivalent to 9.2 ± 0.5 $\mu$g and 11.0 ± 1.3 $\mu$g Mg).

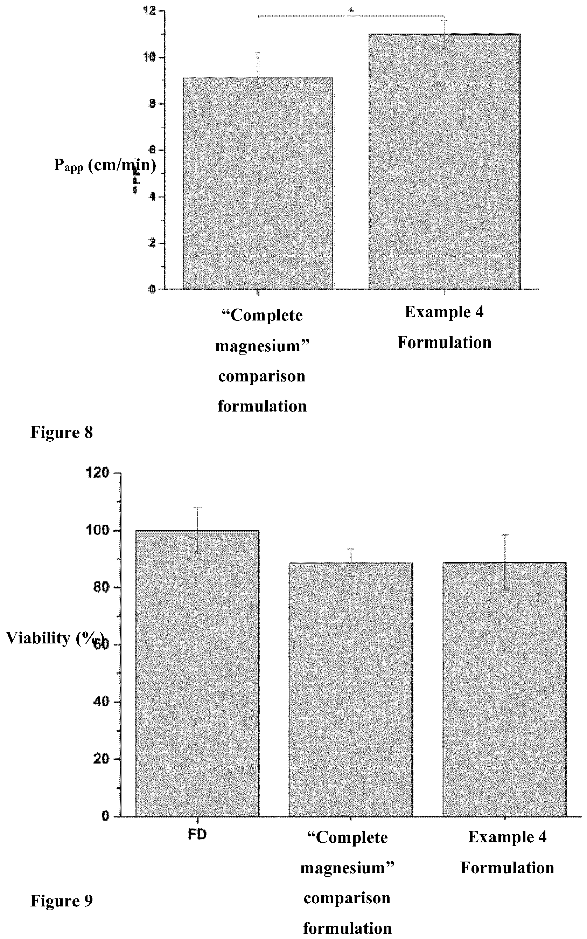

A similar trend was observed for the apparent permeability $P_{app}$ of the magnesium. In fact, the formulation of Example 4 is characterized by a slightly higher apparent permeability than that of the "complete magnesium" formulation (11.0 ± 0.6 cm/min vs 9.1 ± 1.1 cm/min respectively) (Figure 8 and Table 6). This is confirmed by the calculation of the relative permeability obtained by the ratio between $P_{app}$ of the Example 4 formulation and the "complete magnesium" formulation which is 1.2 ± 0.2 (Table 6). A relative permeability value > 1 indicates an increased bioavailability of the Example 4 formulation. Accordingly, the formulation of Example 4 is provided with a better bioavailability of magnesium than the "complete magnesium" formulation.

Table 6 - Bioavailability of magnesium Mg after 3 hours of exposure, expressed as quantity ($\mu$g), percentage (%) with respect to the amount of Mg initially added to the apical compartment, and apparent permeability $P_{app}$ (cm/min). The results are expressed as mean ± standard deviation. LOD (Limit of Detection) = 0.24 $\mu$g/mL.

| Bioavailability | "Complete magnesium" formulation | Example 4 Formulation |
|---|---|---|
| $\mu$g | 11.0 ± 1.3 | 9.2 ± 0.5 |
| % | 1.6 ± 0.2 | 2.0 ± 0.1 |
| $P_{app}$ (x $10^{-5}$ cm/min) | 9.1 ± 1.1 | 11.0 ± 0.6 |
| Relative Permeability ($P_{app}$ Formulation of Example 4 / $P_{app}$ "Complete magnesium" formulation) | 1.2 ± 0.2 | |

### *5.3.4 Impact of the digested Example 4 and "complete magnesium" comparison formulations on intestinal mucosa viability and integrity*

In parallel with absorption, the viability of the intestinal epithelium model was monitored. No significant alterations in the viability of the intestinal epithelium were observed, as expected (Figure 9).

### 5.4 CONCLUSIONS

In light of the results obtained, the formulation of Example 4 is characterized by a higher solubility of magnesium Mg with respect to the "complete magnesium" comparison formulation. Furthermore, the magnesium released from the Example 4 formulation shows better intestinal bioavailability than the "complete magnesium" formulation.

# EP 3 928 764 B1

**Claims**

1. Oral formulation for use in replenishing magnesemia, maintaining muscle, osteo-articular, nervous, and immune function, comprising: ascorbic acid, vitamin $D_3$ and magnesium salts, wherein vitamin $D_3$ is delivered in the form of a micellar dispersion, the magnesium salts contain at least magnesium carbonate and magnesium oxide, and the weight ratio between ascorbic acid and magnesium salts is comprised between 0.2:1 and 1.25:1.

2. Oral formulation for use according to claim 1, wherein the micellar dispersion of vitamin $D_3$ comprises fatty acid sucrose esters.

3. Oral formulation for use according to any one of claims from 1 to 2, wherein the fatty acids sucrose esters are selected from the group consisting of oleic acid sucrose esters, palmitic acid sucrose esters and mixtures thereof.

4. Oral formulation for use according to any one of claims from 1 to 3, wherein vitamin $D_3$ is in amounts ranging from 0.000015 g to 0.005 g.

5. Oral formulation for use according to any one of claims from 1 to 4, wherein the magnesium salts further comprise a magnesium salt selected from magnesium pidolate, magnesium citrate and mixtures thereof.

6. Oral formulation for use according to any one of claims from 1 to 5, wherein the magnesium salts are in amounts ranging from 0.240 g to 1.045 g.

7. Oral formulation for use according to any one of claims from 1 to 6, wherein the ascorbic acid is in amounts ranging from 0.0500 g to 0.300 g.

8. Oral formulation for use according to any one of claims from 1 to 7, in the form of water-dispersible powder.

9. Oral formulation for use according to claim 8, wherein the daily dosage is in amounts ranging from 1.40 g to 5.0 g.

10. Oral formulation for use according to claim 8 or 9, in the form of single-dose sachets or in the form of bottles.

**Patentansprüche**

1. Orale Formulierung zur Verwendung beim Auffüllen von Magnesämie, Aufrechterhalten von Muskel-, Gelenk-, Nerven- und Immunfunktion, umfassend: Ascorbinsäure, Vitamin D3 und Magnesiumsalze, wobei Vitamin D3 in Form einer mizellaren Dispersion zugeführt wird, die Magnesiumsalze mindestens Magnesiumcarbonat und Magnesiumoxid enthalten und das Gewichtsverhältnis zwischen Ascorbinsäure und Magnesiumsalzen zwischen 0,2:1 und 1,25:1 ist.

2. Orale Formulierung zur Verwendung nach Anspruch 1, wobei die mizellare Dispersion von Vitamin D3 Fettsäuresaccharoseester umfasst.

3. Orale Formulierung zur Verwendung nach einem der Ansprüche 1 bis 2, wobei die Fettsäuren-Saccharoseester ausgewählt sind aus der Gruppe, bestehend aus Ölsäure-Saccharoseestern, Palmitinsäure-Saccharoseestern und Gemischen davon.

4. Orale Formulierung zur Verwendung nach einem der Ansprüche 1 bis 3, wobei Vitamin D3 in Mengen im Bereich von 0,000015 g bis 0,005 g ist.

5. Orale Formulierung zur Verwendung nach einem der Ansprüche 1 bis 4, wobei die Magnesiumsalze ferner ein Magnesiumsalz umfassen, ausgewählt aus Magnesiumpidolat, Magnesiumcitrat und Gemischen davon.

6. Orale Formulierung zur Verwendung nach einem der Ansprüche 1 bis 5, wobei die Magnesiumsalze in Mengen in einem Bereich von 0,240 g bis 1,045 g sind.

7. Orale Formulierung zur Verwendung nach einem der Ansprüche 1 bis 6, wobei die Ascorbinsäure in Mengen in einem Bereich von 0,0500 g bis 0,300 g ist.

14

**8.** Orale Formulierung zur Verwendung nach einem der Ansprüche 1 bis 7 in Form eines in Wasser dispergierbaren Pulvers.

**9.** Orale Formulierung zur Verwendung nach Anspruch 8, wobei die tägliche Dosierung in Mengen in einem Bereich von 1,40 g bis 5,0 g ist.

**10.** Orale Formulierung zur Verwendung nach Anspruch 8 oder 9 in Form von Einzeldosisbeuteln oder in Form von Flaschen.

**Revendications**

**1.** Formulation orale destinée à être utilisée dans la reconstitution de la magnésémie, le maintien de la fonction musculaire, ostéo-articulaire, nerveuse et immunitaire, comprenant : de l'acide ascorbique, de la vitamine D3 et des sels de magnésium, dans laquelle la vitamine D3 est administrée sous la forme d'une dispersion micellaire, les sels de magnésium contiennent au moins du carbonate de magnésium et de l'oxyde de magnésium, et le rapport pondéral entre l'acide ascorbique et les sels de magnésium est compris entre 0,2:1 et 1,25: 1.

**2.** Formulation orale destinée à être utilisée selon la revendication 1, dans laquelle la dispersion micellaire de vitamine D3 comprend des esters de sucrose des acides gras.

**3.** Formulation orale destinée à être utilisée selon l'une quelconque des revendications 1 à 2, dans laquelle les esters de sucrose des acides gras sont choisis dans le groupe constitué par : les esters de sucrose d'acide oléique, les esters de sucrose d'acide palmitique et leurs mélanges.

**4.** Formulation orale destinée à être utilisée selon l'une quelconque des revendications 1 à 3, dans laquelle la vitamine D3 est dans des quantités allant de 0,000015 g à 0,005 g.

**5.** Formulation orale destinée à être utilisée selon l'une quelconque des revendications 1 à 4, dans laquelle les sels de magnésium comprennent en outre un sel de magnésium choisi parmi le pidolate de magnésium, le citrate de magnésium et leurs mélanges.

**6.** Formulation orale destinée à être utilisée selon l'une quelconque des revendications 1 à 5, dans laquelle les sels de magnésium sont dans des quantités allant de 0,240 g à 1,045 g.

**7.** Formulation orale destinée à être utilisée selon l'une quelconque des revendications 1 à 6, dans laquelle l'acide ascorbique est dans des quantités allant de 0,0500 g à 0,300 g.

**8.** Formulation orale destinée à être utilisée selon l'une quelconque des revendications 1 à 7, sous forme de poudre dispersible dans l'eau.

**9.** Formulation orale destinée à être utilisée selon la revendication 8, dans laquelle la posologie quotidienne est dans des quantités comprises entre 1,40 g et 5,0 g.

**10.** Formulation orale destinée à être utilisée selon la revendication 8 ou 9, sous forme de sachets monodoses ou sous forme de flacons.

**Figure 1**

**Figure 2**

**Figure 3**

Figure 4

Figure 5

Figure 6

Figure 7

Figure 8

Figure 9

**EP 3 928 764 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020026187 A1

**Non-patent literature cited in the description**

- **ANONYMOUS.** Ultramag Magnesium - Product information sheet. *Pureencapsulations.com,* 01 March 2018, https://www.pureencapsulations.com/media